(19) **Europäisches Patentamt European Patent Office Office européen des brevets**

(11) **EP 3 795 077 B1**

(12) **EUROPEAN PATENT SPECIFICATION**

(45) Date of publication and mention of the grant of the patent:
**01.10.2025 Bulletin 2025/40**

(21) Application number: **20197162.9**

(22) Date of filing: **21.09.2020**

(51) International Patent Classification (IPC):
**A61B 5/346** (2021.01)      **A61B 5/107** (2006.01)

(52) Cooperative Patent Classification (CPC):
**A61B 5/1072; A61B 5/346; A61B 5/7267;**
A61B 5/287; A61B 5/367; A61B 2576/023

(54) **CARDIAC WALL THICKNESS ESTIMATION**

HERZWANDDICKENSCHÄTZUNG

ESTIMATION DE L'ÉPAISSEUR DE PAROI CARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.09.2019 US 201962903851 P**
**10.09.2020 US 202017016611**

(43) Date of publication of application:
**24.03.2021 Bulletin 2021/12**

(73) Proprietor: **Biosense Webster (Israel) Ltd.**
**Yokneam 2066717 (IL)**

(72) Inventors:
• **TSOREF, Liat**
**2066717 Yokneam (IL)**
• **AUERBACH, Shmuel**
**2066717 Yokneam (IL)**
• **AMIT, Matityahu**
**2066717 Yokneam (IL)**
• **AMOS, Yariv Avraham**
**2066717 Yokneam (IL)**
• **SHALGI, Avi**
**2066717 Yokneam (IL)**

(74) Representative: **Carpmaels & Ransford LLP**
**One Southampton Row**
**London WC1B 5HA (GB)**

(56) References cited:
**WO-A1-2018/092071      US-A1- 2018 125 575**
**US-A1- 2019 254 564**

• **MD ZAHANGIR ALOM ET AL: "The History Began from AlexNet: A Comprehensive Survey on Deep Learning Approaches", ARXIV.ORG, CORNELL UNIVERSITY LIBRARY, 201 OLIN LIBRARY CORNELL UNIVERSITY ITHACA, NY 14853, 3 March 2018 (2018-03-03), XP081223589**

# EP 3 795 077 B1

## Description

### CROSS-REFERENCE TO RELATED APPLICATIONS

This application claims the benefit of U.S. Provisional Patent Application 62/903,851, filed September 22, 2019.

### FIELD OF THE INVENTION

The present invention relates generally to processing of electrophysiological signals and ablation, and specifically to estimating properties of cardiac wall tissue using machine learning (ML).

### BACKGROUND OF THE INVENTION

A number of methods may be used to estimate a thickness of a cardiac wall, such as ultrasound, fluoroscopy, and MRI imaging. The estimated wall thickness may be further correlated with electrophysical signals to estimate an injury of the cardiac wall tissue. For example, Takeshi Sasaki et al. describe in "Myocardial Structural Associations with Local Electrograms: A Study of Post-Infarct Ventricular Tachycardia Pathophysiology and Magnetic Resonance Based Non-Invasive Mapping," Circulation Arrhythmia and Electrophysiology, December, 2012; 5(6): 1081-1090, significant associations between left ventricular wall thickness, post infarct scar thickness, and intramural scar location seen in MRI, and local endocardial electrogram bipolar/unipolar voltage, duration, and deflections on electroanatomical mapping.

WO 2018/092071 A1 describes methods for estimating the effectiveness of catheter ablation procedures to form lesions. Lesion effectiveness parameters are received, and effectiveness of a corresponding ablation (optionally planned, current, and/or already performed) is estimated. The estimating is based on use by computer circuitry of an estimator constructed based on observed associations between previously analyzed lesion effectiveness parameters, and observed lesion effectiveness. The estimator is used by application to the received lesion effectiveness parameters.

US 2019/254564 A1 describes a computerized method of tracking a position of an intra-body catheter, comprising: physically tracking coordinates of the position of a distal portion of a physical catheter within the physical body portion of the patient according to physically applied plurality of electrical fields within the body portion and measurements of the plurality of electrical fields performed by a plurality of physical electrodes at a distal portion of the physical catheter; registering the physically tracked coordinates with simulated coordinates generated according to a simulation of a simulated catheter within a simulation of the body of the patient, to identify differences between physically tracked location coordinates and the simulation coordinates; correcting the physically tracked location coordinates according to the registered simulation coordinates; and providing the corrected physically tracked location coordinates for presentation.

US 2018/125575 A1 describes devices and methods for tissue lesion assessment and/or creation based on dielectric properties. In some embodiments, one or more probing frequencies are delivered via electrodes including an electrode in proximity to a tissue (for example, myocardial tissue). Measured dielectric properties (such as impedance properties), optionally together with other known and/or estimated tissue characteristics, are used to determine the lesion state of the tissue. In some embodiments, a developing lesion state is monitored during treatment formation of a lesion (for example, ablation of heart tissue to alter electrical transmission characteristics).

### SUMMARY OF THE INVENTION

An embodiment of the present invention that is described herein after provides a system according to claim 1.

There is additionally provided, in accordance with another embodiment of the present invention, a method according to claim 3.

Further embodiments are defined in the dependent claims.

### BRIEF DESCRIPTION OF THE DRAWINGS

The present invention will be more fully understood from the following detailed description of the embodiments thereof, taken together with the drawings in which:

Fig. 1 is a schematic, pictorial illustration of a catheter-based electrophysiological (EP) sensing, signalanalysis, and IRE ablation system, according to an exemplary embodiment of the present invention;

Fig. 2 is a flow chart of training and use for inference, of a machine learning model to estimate cardiac wall thickness, according to an exemplary embodiment of the present invention;

Fig. 3 illustrates a deep learning algorithm for wall thickness estimation based on autoencoders and a fully connected layer, according to an exemplary embodiment of the present invention; and

Fig. 4 is a schematic illustration of autoencoder architecture used in the deep learning algorithm of Fig. 3, according to an exemplary embodiment of the present invention.

## DETAILED DESCRIPTION OF EMBODIMENTS

OVERVIEW

Cardiac ablation is a common procedure that is used to treat arrhythmias by forming lesions in cardiac tissue of a patient. Such lesions may be formed by irreversible electroporation (IRE), or other types of ablative energy, such as radiofrequency (RF), both of which can be applied using a catheter. In IRE ablation, the catheter is maneuvered such that electrodes disposed on a distal end of the catheter are in contact with, or in close proximity to, the tissue. Then high voltage bipolar pulses are applied between the electrodes, and strong electric field pulses produced in tissue cause cell death and lesion production. In RF ablation, an alternating RF current is applied to the tissue by one or more electrodes, causing cell death by heat.

In to order be effective, a tissue ablation has to be transmural, i.e., to penetrate into the depth of the tissue. However, "over-ablation" may create undesired damage to tissue (including, rarely, perforation of the cardiac wall) or harm to adjacent structures such as the esophagus which may be behind the cardiac tissue. Hence, it is important to be able to assess cardiac wall thickness (e.g. atrial or ventricular wall) just before and/or during ablation, in order to use optimal ablation parameters during the procedure.

Different imaging modalities may be employed to assess cardiac wall thickness, including magnetic resonance imaging (MRI), computerized tomography (CT), ultrasound, and more. However, using these modalities adds to the cost and the complexity of the procedure. Moreover, the spatial resolution of these modalities may be on the order of the tissue thickness, which may yield less accurate estimation of the actual wall thickness during ablation.

Embodiments of the present invention that are described hereinafter provide systems and methods to estimate the thickness of a cardiac wall, i.e., an atrial wall or a ventricular wall, just before and/or during ablation with limited up to date information at hand. In some embodiments, a machine learning (ML) model, such as an artificial neural network (ANN), is provided to allow for this estimation using only EP data, such as multi-channel (e.g., 12-lead) body-surface electrocardiogram (ECG), and intra-cardiac electrograms (EGM) acquired by electrodes of a catheter just before or during the ablation procedure.

In some embodiments, the ML model is trained using data comprising EP data (multi-channel ECGs and EGMs - the later also known as intra-cardiac ECG (IcECG)), patient medical history, and 3D location information of the data collected. The model is optimized to gain, via the training, predictive power of the wall thickness using ground truth data, such as atrial/ventricular wall thickness assessed by imaging modalities such as ultrasound, CT, MRI or similar imaging modalities.

The training data may also include (e.g., incorporate), along with the above data items, data collected after the start of ablation, and further initial ablation data, such as a temperature rise profile and/or an impedance change during ablation. (The temperature rise profile is detectable very quickly, typically 10 mSec or 100 mSec, in an ablation that typically takes between four (4) to sixty (60) seconds).

Just before and/or during a new ablation procedure (i.e., during inference), the model uses, as noted above, only EP data, including ECG and EGMS and subsequent ablation data (i.e., any subsequent data acquired during the ablation procedure) for a specific patient, in further assessing wall thickness of the patient.

While the ANN model is used here as an example, a person skilled in the art may choose from among other ML models available for use, such as decision tree learning, support vector machines (SVM), and Bayesian networks. ANN models include, for example, convolutional NN (CNN), autoencoder, and probabilistic neural network (PNN). Typically, the one or more processors used (collectively named hereinafter "processor") are programmed in software containing a particular algorithm that enables the processor to conduct each of the processor-related steps and functions outlined above. Typically, the training is done using a computing system comprising multiple processors, such as graphics processing units (GPU) or tensor processing units (TPU). However, any of these processors may also be central processing units (CPUs).

The ability to assess cardiac wall thickness just before ablation, as well as during ablation, i.e., in real time, based on at least part of the data referred to above using an ML algorithm, allows a simple assessment of cardiac wall thickness, and may lead to a more accurate ablation time, and typically to an improvement in outcome of the ablation procedure as well.

SYSTEM DESCRIPTION

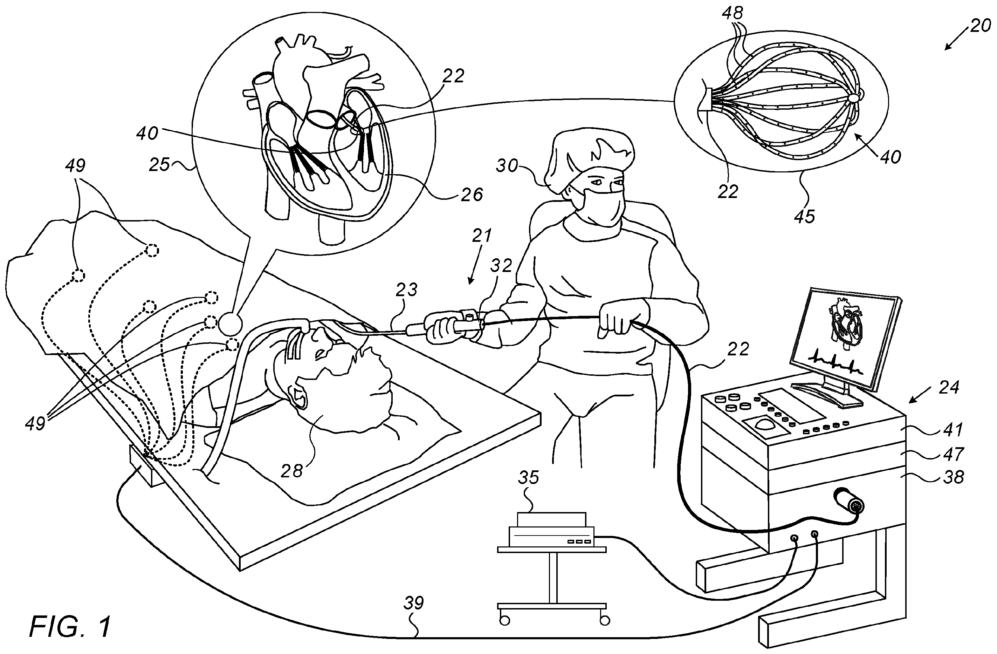

Fig. 1 is a schematic, pictorial illustration of a catheter-based electrophysiological (EP) sensing, signalanalysis, and IRE ablation system 20, according to an exemplary embodiment of the present invention. System 20 may be, for example, a CARTO® 3 system, produced by Biosense-Webster. As seen, system 20 comprises a catheter 21, having a shaft 22 that is navigated by a physician 30 into a heart 26 (inset 25) of a patient 28. In the pictured example, physician 30 inserts shaft 22 through a sheath 23, while manipulating shaft 22 using a manipulator 32 near the proximal end of the catheter.

In the embodiment described herein, catheter 21 may be used for any suitable diagnostic purpose and/or tissue ablation, such as electrophysiological mapping of heart 26 and IRE ablation, respectively. An ECG recording instrument 35 may receive various types of ECG signals sensed by system 20 during the process.

As shown in inset 25, a distal end of shaft 22 of catheter 21 is fitted with a multi-electrode basket catheter 40. Inset 45 shows an arrangement of multiple electrodes 48 of basket catheter 40. The proximal end of catheter 21 is connected to a control console 24, to transmit, for example, electrograms acquired by electrodes 48.

Console 24 comprises a processor 41, typically a general-purpose computer, with suitable front end and interface circuits 38 for receiving EP signals (e.g., ECGs and EGMS) as well as non-EP signals (such as position signals) from electrodes 48 of catheter 21. For this purpose, processor 41 is connected to electrodes 48 via wires running within shaft 22. Interface circuits 38 are further configured to receive ECG signals, such as from a 12-lead ECG apparatus that can be ECG recording instrument 35, as well as non-ECG signals from surface body electrodes 49. Typically, electrodes 49 are attached to the skin around the chest and legs of patient 28. Processor 41 is connected to electrodes 49 by wires running through a cable 39 to receive signals from electrodes 49.

Four of surface body electrodes 49 are named according to standard ECG protocols: RA (right arm), LA (left arm), RL (right leg), and LL (left leg). A Wilson Central Terminal (WCT) may be formed by three of the four named body surface electrodes 49, and a resulting ECG signal, $V_{WCT}$, is received by interface circuits 38.

During an EP mapping procedure, the locations of electrodes 48 are tracked while they are inside heart 26 of the patient. Such tracking may be performed using the Active Current Location (ACL) system, made by Biosense-Webster, which is described in US Patent No. 8,456,182.

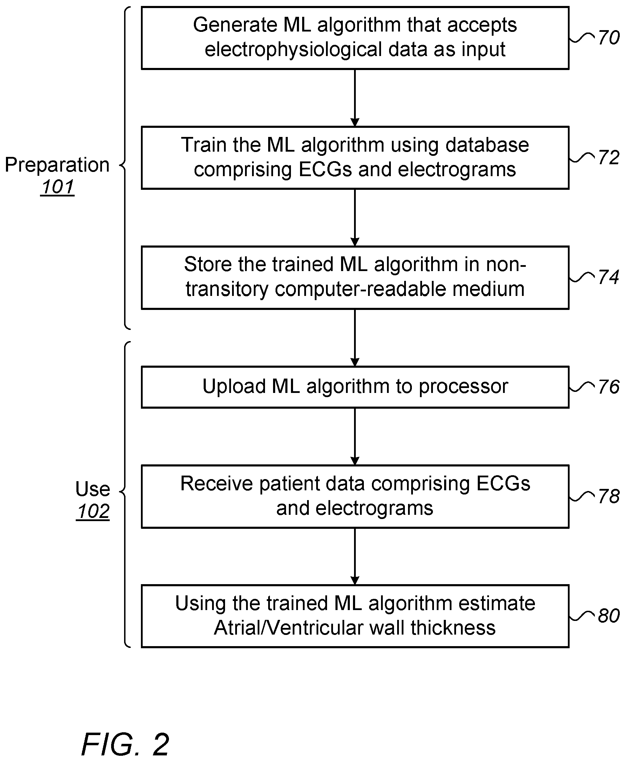

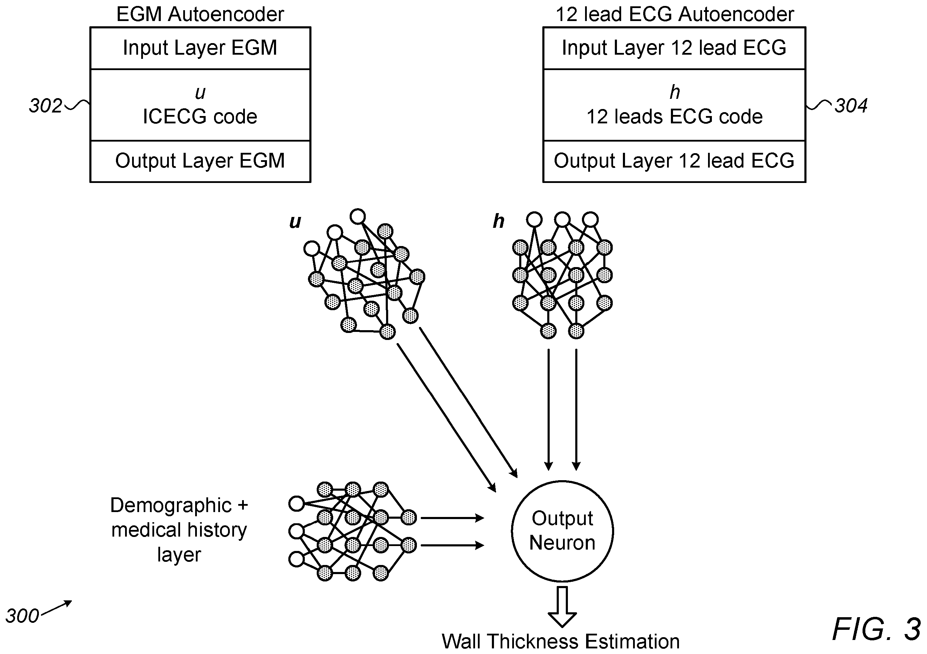

The processor may thus associate any given signal received from electrodes 48, such as EGMs, with the location at which the signal was acquired. Processor 41 uses information contained in these signals to construct an EP map, such as a local activation time (LAT) map, to present on a display. In the shown embodiment, using an algorithm comprising an ML algorithm applied to EP and other data, as described in Figs. 2 and 3, processor 41 estimates cardiac wall thickness.

To perform IRE ablation, electrodes 48 are connected (e.g., switched) to an IRE pulse generator 47 comprising a processor-controlled switching circuitry (e.g., an array of relays, not shown) in console 24. Using the wallthickness information, processor 41, or the physician, may select which electrodes to connect to pulse-generator 47 to apply IRE pulses (via the switching circuitry).

During RF ablation, initial and subsequent ablation data comprise at least one of the following: an IRE energy profile, a temperature rise, and a change of impedance. They may be used in further assessing (e.g., in real time) wall thickness of the patient, as described in Fig. 2.

Processor 41 is typically programmed in software to carry out the functions described herein. The software may be downloaded to the processor in electronic form, over a network, for example, or it may, alternatively or additionally, be provided and/or stored on non-transitory tangible media, such as magnetic, optical, or electronic memory. In particular, processor 41 runs a dedicated algorithm as disclosed herein, included in Fig. 3, that enables processor 41 to perform the disclosed steps, as further described below.

ECG-BASED CARDIAC WALL THICKNESS ESTIMATION USING ML

Fig. 2 is a flow chart of training, and use for inference, of a machine learning model to estimate cardiac wall thickness, according to an exemplary embodiment of the present invention.

The algorithm, according to the presented exemplary embodiment, is divided into two parts: algorithm preparation 101 and algorithm use 102.

Algorithm preparation carries out a process that begins at ML modeling step 70, to generate an ML algorithm for estimating cardiac wall thickness.

Next, using a database including ECGs and EGMs, a processor trains the algorithm (e.g. the ANN and preprocessing parts), at an ML algorithm training step 72. In step 72, the processor uses training data, including ground truth data, to train the ML model. Training data is formed from:

1. Multi-channel (e.g., 12-lead) ECG data
2. Electrograms with 3D information of the cardiac tissue collection location
3. Anatomical location of each intracardiac electrode - Based on another ML model / 3D segmentation of the atria using MFAM).
4. Diagnostic catheter details

5. Patient demographic information, (for example, gender, age, height, weight)
6. Patient medical history

Ground truth data is formed from:

7. Atrial/ventricular wall thickness assessed by imaging modalities such as ultrasound, CT, MRI or similar imaging modalities.

Additional training data may also include an ablation transmitting energy profile, as well as ablation related parameters such as a temperature rise and/or an impedance change and/or an elasticity change and/or a stiffness change during ablation. The data items #1 - #6 above, while collected after ablation begins, and/or the additional collected training data, are collectively termed herein "ablation data."

The algorithm preparation ends with storing the trained model in a non-transitory computer-readable medium, such as a disc on key (memory stick), at a trained model storing step 74. In alternative embodiments, the model is sent in advance, and its optimized parameters (such as weights of an ANN) are sent separately after training.

Algorithm use 102 carries out a process that begins at algorithm uploading step 76, during which a user uploads either an entire ML model or its optimized parameters (e.g., weights) to a processor. Next, the processor, such as processor 28, receives patient inference data, for example, the aforementioned ECGs and EGMs from electrodes 49 and 48, respectively, at patient data receiving step 78.

Next, using the trained ML model for inference, the processor inputs data from a selected patient to the trained model, and implements an algorithm on the model so that the model is able to output an atrial or a ventricular wall thickness of the patient only from limited data available, such as the aforementioned EP data, at cardiac wall thickness estimation step 80. After being installed on the processor, the trained model may be used with multiple patients.

In some embodiments, the NN model outputs a statistical distribution of thicknesses, and a peak of the distribution may be selected in a subsequent step, i.e., beyond those included in the NN model, to determine the most likely wall thickness value.

The example flow chart shown in Fig. 2 is chosen purely for the sake of conceptual clarity. The present embodiment may also comprise additional steps of the algorithm, such as receiving indications of the degree of physical contact of the electrodes with diagnosed tissue. This and other possible steps are omitted from the disclosure herein purposely in order to provide a more simplified flow chart.

ML ALGORITHM DESCRIPTION

Fig. 3 illustrates a deep learning algorithm 300 for wall thickness estimation based on autoencoders and a fully connected layer, according to an exemplary embodiment of the present invention. The method comprises providing a deep learning supervised framework for the estimation, and uses electrograms, 12-lead ECG, anatomical data, catheter details, demographic data of a patient, and the medical history of the patient, (corresponding to training data items #1 - #6 above). The method may also use a temperature rise and/or an impedance change during ablation.

In the method, two autoencoders 302 and 304 (described in more detail below) are applied to perform dimensionality reduction to a set of features from the 12-lead ECG and/or from the intracardiac ECG. The method uses a fully connected layer based on those features and based on medical history information including, but not limited to, a NYHA (New York Heart Association) score, a CHA2DS2-VASc score, and an AF duration, as well as demographic data (e.g. age, gender, height and weight). A regression analysis is then performed in order to estimate the thickness of the cardiac wall.

As stated above, the method uses two autoencoders 302 and 304. An autoencoder comprises two parts: an encoder and a decoder. The encoder maps an input (in Fig. 3, an ECG signal and/or an EGM signal) to a hidden representation (h or u, respectively) via a nonlinear transformation. The decoder then maps the hidden representation back to reconstructed data via another nonlinear transformation. Equations 1 and 2 represent the mappings:

$$\text{Eq. 1} \qquad h = f(ECG, \theta_{encoder}), \ ECG' = g(h, \theta_{decoder}),$$

$$\text{Eq. 2} \qquad u = f(EGM, \emptyset_{encoder}), \ EGM' = g(u, \emptyset_{decoder}),$$

Where $\theta_{encoder}, \theta_{decoder}$ are weights for the ECG signal reconstruction, and $\emptyset_{encoder}, \emptyset_{decoder}$ are weights for the EGM signal reconstruction.

The same network architecture is used for ECG and EGM reconstruction, so that the nonlinear functions f and g are substantially the same. Using a minimize L2 normalization function between a set of autoencoders provides a set of $\theta_{encoder}, \theta_{decoder}$ weights for ECG signal reconstruction and a set of $\emptyset_{encoder}, \emptyset_{decoder}$ weights for EGM reconstruction.

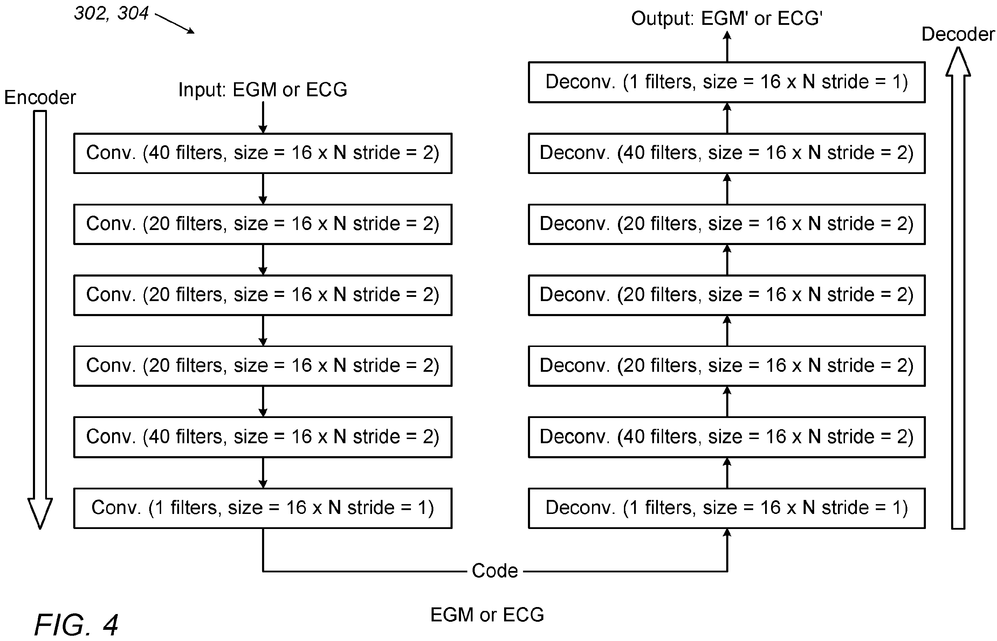

Fig. 4 is a schematic illustration of autoencoder architecture used in the deep learning algorithm of Fig. 3, according to an exemplary embodiment of the present invention. In particular, the autoencoder architecture is used for compressing and learning feature space of electrograms and/or 12-lead ECG. Each autoencoder is implemented using a fully connected convolutional neural network (FCN) of an encoder and a decoder with a predefined number of layers, as shown in the figure. In the encoder, the size of EGM/ECG signals is reduced, and the signals are encoded into low dimensional features. The decoder attempts to reconstruct an output depending on the low dimensional features. Embodiments of the invention employ rectifier linear units (ReLUs) as activation functions for hidden layers. There is no activation function for the output layer in the FCN model. In addition, each hidden layer undergoes batch normalization.

The encoder contains a series of layers, where each individual layer is composed of a convolutional layer, a batch normalization layer, and an activation layer. The input layer is defined by the original signals with a size of 1024×N, where N represents the number of inputted channels. Thus N=12 for a 12-lead ECG, and for intracardiac ECG signals N corresponds to the number of electrodes on the catheter-acquiring signals. For example, N=20 is used for a PentaRay or Lasso catheter, and N=64 used for the basket catheter of Fig. 1. It will be understood that the catheters referred to hereinabove are examples, and that the scope of the present invention comprises any cardiac catheter.

A convolutional process with 40 filters of size 16×N and a stride of 2 is applied on the first layer. The next three convolutional layers all have 20 filters of size 16×N with a stride of 2. Then, the next layer consists of 40 filters of size 16×N with a stride of 2. The last layer has one filter of size 16×1 with a stride of 1. The downsampling process is achieved using a stride of 2. Through the encoding process, a 32×N dimensional feature map is obtained. This feature map also represents the compressed data and is 32 times smaller than the original data size.

The decoder part of the autoencoder is inversely symmetric to the encoder. Here, the deconvolutional layers proceed to up-sample the feature maps so as to recover structural details. As for the output layer, a final deconvolutional layer with one filter of size 16×N and a stride of 1 produces the output signal.

Returning to Fig. 3, hidden representations h and u, patient medical history information (NYHA score, CHA2DS2-VASc score, AF duration and persistent AF duration) and patient demographic data (age, gender, height and weight) serve as a feature space (light gray circles in Fig. 3) for a fully connected neural network with four hidden layers (dark gray circles). In some embodiments, the feature space also comprises at least one of a temperature rise and an impedance change inputs.

The outputs from the hidden layers are then inserted into an output neuron which estimates the wall thickness of the heart.

The entire network is trained using a backpropagation algorithm that attempts to minimize an L2 regularization function, shown in equation 3.

$$\text{Eq. 3} \qquad J(\varphi) = \sum_i \left\| WT_i - WT_i{}' \right\|^2 - \beta \left\| \varphi \right\| \ ,$$

where $J(\varphi)$ is a loss function, $WT_i$ represents an atrial/ventricular wall thickness taken from ultrasound or CT, MRI, or similar imaging modalities of a subject i, $WT_i{}'$ is the cardiac wall thickness estimated based on the suggested method, $\varphi$ is a weight of a fully connected layer, and $\beta$ is a regularization parameter. In a disclosed embodiment $\beta$ is set at 0.01.

The backpropagation algorithm is performed to minimize the loss function $J(\varphi)$.

In exemplary embodiments of the invention a deep learning regressor for cardiac wall thickness is obtained after learning the optimal values (in an L2 regularization sense) of the set of parameters $\varphi$, $h$, $u$, $\theta_{encoder}$, $\theta_{decoder}$, $\varnothing_{encoder}$, $\varnothing_{decoder}$.

The disclosed embodiment provides, by way of example only, specific numbers, such as number of filters. In general, such numbers may be modified. While the description above refers to an ablation procedure, and to measuring a tissue wall thickness for the procedure, it will be appreciated that the description may be adapted, *mutatis mutandis,* to measuring tissue wall thickness absent an ablation procedure. Thus, the scope of the present invention comprises devices used in cardiac procedures with or without an ablation procedure. The scope of the present invention comprises methods without an ablation procedure.

It will thus be appreciated that operating the algorithm described above enables the processor to approximate the thickness of a cardiac wall. The value may be incorporated into a GUI of an ablation system. Alternatively, or additionally, the thickness value may be presented as a number on a "wall" drawing, or the wall thickness may be displayed graphically according to a scale of the heart image presented on an ablation system display.

It will consequently be appreciated that the embodiments described above are cited by way of example, and that the present invention is not limited to what has been particularly shown and described hereinabove. Although the embodiments described herein mainly address cardiac diagnostic applications, the methods and systems described herein can also be used in other cardiac medical applications that require estimation of cardiac wall thickness.

It will be therefore further appreciated that the embodiments described above are cited by way of example, and that the present invention is not limited to what has been particularly shown and described hereinabove. Rather, the scope of the present invention is defined by the claims.

## Claims

1. A system (20) for estimating properties of cardiac wall tissue, the system (20) comprising:

   an interface (38), configured to receive a plurality of electrophysiological (EP) measurements performed in a heart of a patient; and
   a processor (41), configured to estimate a wall thickness at a specified location of the heart based on the EP measurements using a trained machine learning (ML) model,
   wherein one or more of the EP measurements comprise intra-cardiac electrograms (EGMs) and one or more of the EP measurements comprise body-surface electrocardiograms (ECGs), and
   the ML model comprises a first autoencoder configured to operate on the EGMs and a second autoencoder configured to operate on the ECGs, the first and second autoencoders each comprising an encoder coupled to a decoder.

2. The system according to claim 1, wherein the processor is configured to estimate the wall thickness using the model, the model defined over the EP measurements, wherein the interface is further configured to receive results of an ablation procedure applied at the specified location of the heart, and wherein the processor is configured to refine the model based on the results of the ablation procedure applied at the specified location of the heart.

3. A method for estimating properties of cardiac wall tissue, the method comprising:

   receiving a plurality of electrophysiological (EP) measurements performed in a heart of a patient; and
   estimating a wall thickness at a specified location of the heart based on the EP measurements using a trained machine learning (ML) model,
   wherein one or more of the EP measurements comprise intra-cardiac electrograms (EGMs) and wherein one or more of the EP measurements comprise body-surface electrocardiograms (ECGs), and
   the ML model comprises a first autoencoder configured to operate on the EGMs and a second autoencoder configured to operate on the ECGs, the first and second autoencoders each comprising an encoder coupled to a decoder.

4. The system (20) according to claim 1 or the method according to claim 3, wherein the EP measurements further comprise respective locations in the heart at which the EGMs were acquired.

5. The system according to claim 2 wherein the results of the ablation procedure comprise one or more of (i) a temperature rise associated with the ablation procedure, and (ii) a change in tissue impedance associated with the ablation procedure.

## Patentansprüche

1. System (20) zum Schätzen von Eigenschaften von Herzwandgewebe, das System (20) umfassend:

   eine Schnittstelle (38), die konfiguriert ist, um eine Vielzahl von elektrophysiologischen (EP) Messungen zu empfangen, die in dem Herzen eines Patienten durchgeführt werden; und
   einen Prozessor (41), der konfiguriert ist, um eine Wanddicke an einer spezifizierten Stelle des Herzens basierend auf den EP-Messungen unter Verwendung eines trainierten maschinellen Lernmodells (ML) zu schätzen,
   wobei eine oder mehrere der EP-Messungen intrakardiale Elektrogramme (EGMs) und eine oder mehrere der EP-Messungen Körperoberflächen-Elektrokardiogramme (EKGs) umfassen, und
   das ML-Modell einen ersten Autoencoder, der konfiguriert ist, um auf den EGMs zu arbeiten, und einen zweiten Autoencoder, der konfiguriert ist, um auf den EKGs zu arbeiten, umfasst, der erste und der zweite Autoencoder jeweils umfassend einen Encoder, der mit einem Decoder gekoppelt ist.

2. System nach Anspruch 1, wobei der Prozessor konfiguriert ist, um die Wanddicke unter Verwendung des Modells zu schätzen, wobei das Modell über die EP-Messungen definiert ist, wobei die Schnittstelle ferner konfiguriert ist, um Ergebnisse einer Ablationsprozedur, das an der spezifizierten Stelle des Herzens angewendet wird, und wobei der Prozessor konfiguriert ist, um das Modell basierend auf den Ergebnissen der Ablationsprozedur, das an der spezifizierten Stelle des Herzens angewendet wird, zu verfeinern.

3. Verfahren zum Schätzen der Eigenschaften von Herzwandgewebe, das Verfahren umfassend:

Empfangen einer Vielzahl von elektrophysiologischen (EP) Messungen, die an dem Herzen eines Patienten durchgeführt werden; und

Schätzen einer Wanddicke an einer spezifizierten Stelle des Herzens basierend auf den EP-Messungen unter Verwendung eines trainierten maschinellen Lernmodells (ML), wobei eine oder mehrere der EP-Messungen intrakardiale Elektrogramme (EGMs), und wobei eine oder mehrere der EP-Messungen Körperoberflächen-Elektrokardiogramme (EKGs) umfassen, und das ML-Modell einen ersten Autoencoder, der konfiguriert ist, um auf den EGMs zu arbeiten, und einen zweiten Autoencoder, der konfiguriert ist, um auf den EKGs zu arbeiten, umfasst, der erste und der zweite Autoencoder jeweils umfassend einen Encoder, der mit einem Decoder gekoppelt ist.

4. System (20) nach Anspruch 1 oder Verfahren nach Anspruch 3, wobei die EP-Messungen ferner jeweilige Stellen in dem Herzen, an denen die EGMs erfasst wurden, umfassen.

5. System nach Anspruch 2, wobei die Ergebnisse der Ablationsprozedur eines oder mehrere umfassen von (i) einem Temperaturanstieg, der mit der Ablationsprozedur verknüpft ist, und (ii) einer Änderung in der Gewebeimpedanz, die mit der Ablationsprozedur verknüpft ist, umfassen.

**Revendications**

1. Système (20) d'estimation des propriétés du tissu de la paroi cardiaque, le système (20) comprenant :

une interface (38), configurée pour recevoir une pluralité de mesures électrophysiologiques (EP) effectuées dans un cœur d'un patient ; et

un processeur (41), configuré pour estimer une épaisseur de la paroi à un endroit précis du cœur sur la base des mesures EP à l'aide d'un modèle d'apprentissage machine (ML) entraîné, dans lequel une ou plusieurs des mesures EP comprennent des électrogrammes intra-cardiaques (EGM) et une ou plusieurs des mesures EP comprennent des électrocardiogrammes (ECG) à la surface du corps, et le modèle ML comprend un premier autoencodeur configuré pour fonctionner sur les EGM et un second autoencodeur configuré pour fonctionner sur les ECG, le premier et le second autoencodeurs comprenant chacun un encodeur couplé à un décodeur.

2. Système selon la revendication 1, dans lequel le processeur est configuré pour estimer l'épaisseur de la paroi à l'aide du modèle, le modèle étant défini sur les mesures EP, dans lequel l'interface est en outre configurée pour recevoir les résultats d'une procédure d'ablation appliquée à l'emplacement spécifié du cœur, et dans lequel le processeur est configuré pour affiner le modèle sur la base des résultats de la procédure d'ablation appliquée à l'emplacement spécifié du cœur.

3. Procédé d'estimation des propriétés du tissu de la paroi cardiaque, le procédé comprenant :

la réception d'une pluralité de mesures électrophysiologiques (EP) effectuées dans un cœur d'un patient ; et l'estimation d'une épaisseur de la paroi à un endroit précis du cœur sur la base des mesures EP à l'aide d'un modèle d'apprentissage automatique (ML) entraîné, dans lequel une ou plusieurs des mesures EP comprennent des électrogrammes intra-cardiaques (EGM) et dans lequel une ou plusieurs des mesures EP comprennent des électrocardiogrammes (ECG) à la surface du corps, et le modèle ML comprend un premier autoencodeur configuré pour fonctionner sur les EGM et un second autoencodeur configuré pour fonctionner sur les ECG, le premier et le second autoencodeurs comprenant chacun un encodeur couplé à un décodeur.

4. Système (20) selon la revendication 1 ou le procédé selon la revendication 3, dans lequel les mesures EP comprennent en outre les emplacements respectifs dans le cœur où les EGM ont été acquis.

**5.** Système selon la revendication 2, dans lequel les résultats de la procédure d'ablation comprennent un ou plusieurs des éléments suivants : (i) une augmentation de la température associée à la procédure d'ablation, et (ii) une modification de l'impédance du tissu associée à la procédure d'ablation.

FIG. 1

Preparation
*101*

| Generate ML algorithm that accepts electrophysiological data as input | 70 |

↓

| Train the ML algorithm using database comprising ECGs and electrograms | 72 |

↓

| Store the trained ML algorithm in non-transitory computer-readable medium | 74 |

↓

Use
*102*

| Upload ML algorithm to processor | 76 |

↓

| Receive patient data comprising ECGs and electrograms | 78 |

↓

| Using the trained ML algorithm estimate Atrial/Ventricular wall thickness | 80 |

*FIG. 2*

EGM Autoencoder

| Input Layer EGM |
| --- |
| $u$<br>ICECG code |
| Output Layer EGM |

302

12 lead ECG Autoencoder

| Input Layer 12 lead ECG |
| --- |
| $h$<br>12 leads ECG code |
| Output Layer 12 lead ECG |

304

$u$

$h$

Demographic +
medical history
layer

Output
Neuron

300

Wall Thickness Estimation

*FIG. 3*

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62903851
- WO 2018092071 A1
- US 2019254564 A1
- US 2018125575 A1
- US 8456182 B

**Non-patent literature cited in the description**

- **TAKESHI SASAKI et al.** Myocardial Structural Associations with Local Electrograms: A Study of Post-Infarct Ventricular Tachycardia Pathophysiology and Magnetic Resonance Based Non-Invasive Mapping. *Circulation Arrhythmia and Electrophysiology*, December 2012, vol. 5 (6), 1081-1090